# EUROPEAN PATENT SPECIFICATION

(11) **EP 1 907 360 B1**
(45) Date of publication and mention of the grant of the patent: **30.03.2011**
(21) Application number: 05783707.2
(22) Date of filing: 09.09.2005
(51) Int. Cl.: C07D 213/127, C07D 213/40

(54) **IMPROVED PROCESS FOR THE MANUFACTURE OF FLECAINIDE**
VERBESSERTES VERFAHREN ZUR HERSTELLUNG VON FLECAINID
PROCÉDÉ AMÉLIORÉ DE FABRICATION DE LA FLÉCAÏNIDE

(30) Priority: 04.07.2005 IN MU07892005
(43) Date of publication of application: 09.04.2008
(73) Proprietor: Glade Organics Private Limited, Mumbai 400 054 (IN)
(72) Inventor: CHAWLA, Harmander Pal Singh, Chandigarh - 160 047 (IN); CHOWDHARY, Anil shankar, E-202 Kasturi Apartment, Bodakdev, Ahmedabad 380015 (IN); LIMBAD, Piyush Bhikhubhai, J-5, Kadambari Apt., Satelite, Ahmedabad 380015 (IN); JHA, Rajesh Jagannathbhai, Bokaro Jharkhand 827006 (IN); JOSHI, Vipul Narbheshankar, Kadia Plot, Porbandar 360575 (IN)
(74) Representative: Bassil, Nicholas Charles
(86) International application number: PCT/IB2005/002682
(87) International publication number: WO 2007/003982

(56) References cited:
- WO-A1-99/02498
- WO-A1-02/066413
- WO-A2-02/04419
- BANITT E.H. ET AL.: 'Antiarrhythmics. 2. Synthesis and Antiarrhythmic Activity of N-(Piperidylalkyl)trifluoroethoxybenzamides ' JOURNAL OF MEDICINAL CHEMISTRY vol. 20, no. 6, 1977, pages 821 - 826, XP002215769

## Description

### BACKGROUND OF THE INVENTION

The present invention relates to an improved process for the manufacture of *N*-(pyridin-2-ylmethyl) 2,5-bis(2,2,2-trifluoroethoxy)benzamide II, which is the penultimate intermediate in the manufacture of N-(2-piperidinylmethyl)-2,5-bis(2,2,2-trifluoroethoxy) benzamide acetate (flecainide acetate) of the formula I.

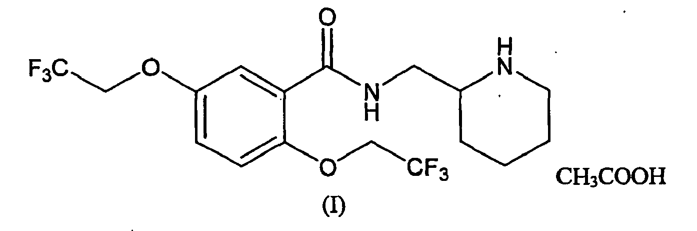

Flecainide acetate (I) is a widely used class 1c antiarrhythmic agent. It is indicated for various types of arrhythmias. It is used to regulate the rate and rhythm of the heart. The heart's pumping action is controlled by electrical signals that pass through the heart muscle. The electrical signals cause the two pairs of heart chambers (left and right arteria and ventricles) to contract in a regular manner to produce regular heartbeats. If the electrical activity in the heart is disturbed for any reason, irregular heartbeats (arrhythmias) of various types can result. These can seriously undermine the pumping action of the heart and result in inefficient blood circulation around the body. Flecainide helps to treat arrhythmias by decreasing the sensitivity of the heart muscle cells to electrical impulses. This regulates the electrical conduction in the heart muscle and reduces disturbances in the heart rhythm. Several processes for the manufacture of the same are reported.

A key intermediate in the synthesis of the compound of the formula I is the compound of the formula II. This intermediate is also the object of the invention of several earlier patents.

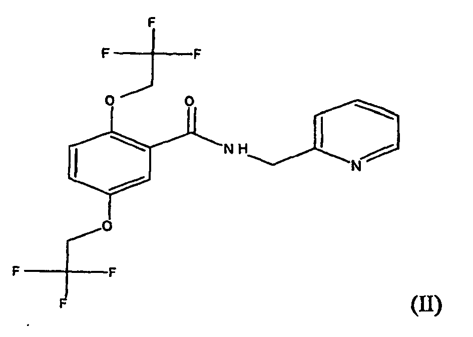

### PRIOR ART

The United States Patent No. 3,900,481 relates to the manufacture of certain compounds in which a carbon atom of a pyrrolidine or piperidine is bonded directly or through a methylene group to the nitrogen of a substituted benzamido group and their pharmaceutically acceptable salts are active as antiarrhythmic agents.

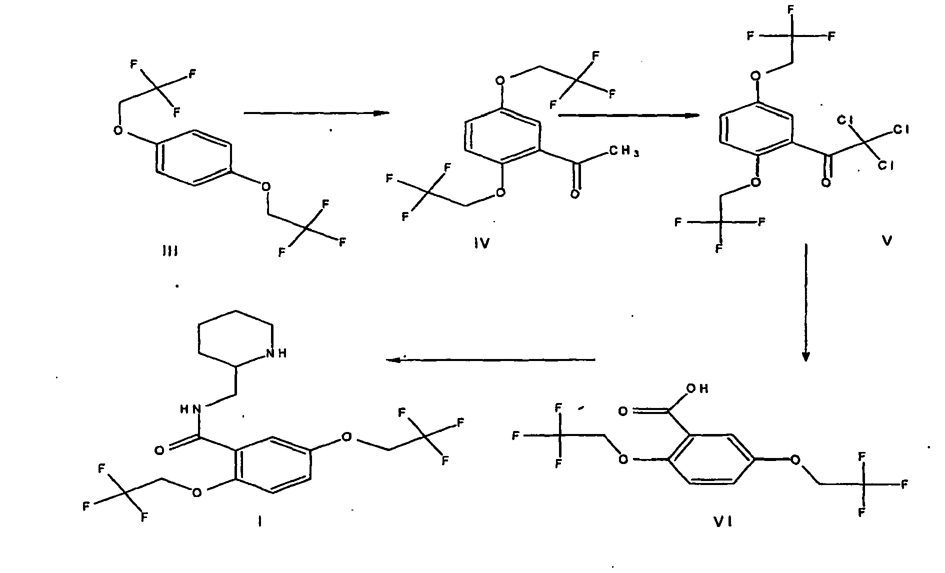

Herein, flecanide was prepared by converting 1,4-R₂C₆H₄ (R= halogen, OH) into 1,4-(F₃CCH₂O)₂C₆H₄ (III), which was acetylated to give 2,5-(F₃CCH₂O)₂C₆H₃COMe (IV), which was then chlorinated to give 2,5-(F₃CCH₂O)₂C₆H₃COCCl₃ (V), which was finally hydrolyzed to (F₃CCH₂O)₂C₆H₃CO₂H (VI) and was converted to acid chloride followed by reaction with 2-aminomethylpiperidine to give I. This process utilizes hazardous chemicals and intermediates, which are commercially not available from multiple sources and is hence not of commercial importance. Besides this process gives the compound in low purities due to coupling occurring at the piperidinyl nitrogen and is hence not economical and commercially viable.

The US patents 4,097,481 and US 4,617,396 deal with tertiary amide derivatives of pyrrolidine and piperidine and process for their preparation but essentially having a similar strategy for amidification as described for US 3,900,481. These processes suffer similarly as detailed above.

The US patent 4,642,384 relates to processes for the preparation of intermediates such as 2,5-bis(2,2,2-trifluoroethoxy)acetophenone, 2,5-bis(2,2,2-trifluoroethoxy)α,α-dichloroacetophenone, 2,5-bis(2,2,2-trifluoroethoxy)α,α,α-trichloroacetophenone and subsequent condensation with 2-aminomethylpyridine followed by catalytic hydrogenation to afford Flecainide acetate of the formula I. This process is again not advantageous on an industrial scale.

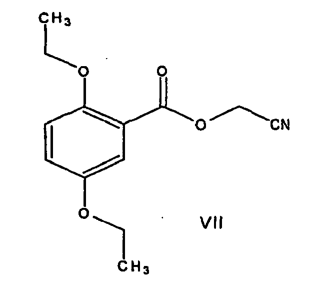

The patent US 6,316,627 provides with a process for preparation of the title compound characterized in that the product namely, 2,5-bis(2,2,2-trifluoroethoxy)benzoic acid was reacted with XCH₂CN to form a cyanomethyl ester derivative (VII) and was converted to flecainide by reacting with RCH₂NH₂ (where R denotes pyridyl group) followed by catalytic hydrogenation. This patent makes use of expensive haloalkyl nitriles for forming the intermediate and is thus far from satisfactory.

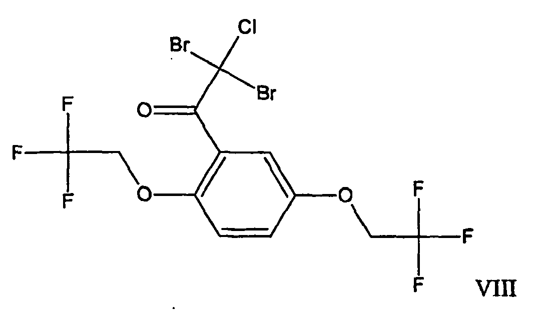

The patent US 6.458,957 related to the use of α,α-dibromo-α-chloroacetophenone compounds (VIII), more particularly 2,5-bis(2,2,2-trifluoroethoxy)-α,α-dibromo-α-chloroacetophenone as intermediates for preparing Flecainide. The process comprises of converting the α-chloro acetophenone to an α,α-dibromo-α-chloroacetophenone derivative and reacting the same with a primary or secondary amine. The process is further characterized in that when the amine is 2-(aminomethyl)pyridine it forms 2,5-bis(2,2,2 trifluoroethoxy)-N-(2-pyridylmethyl)benzamide and reducing the same with hydrogen affords 2,5-bis(2.2,2 trifluoroethoxy)-N-(2-piperidylmethyl)benzamide. This process involves bromination and is hence unsatisfactory from an ecological point of view.

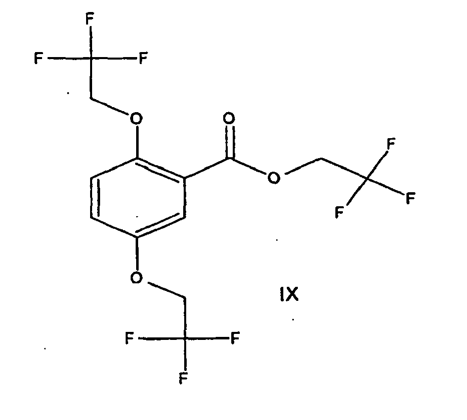

The patent US 6.599,992 relates to a process for the preparation of flecainide comprising of synthesis of the key intermediate 2',2',2'-trifluoroethanol 2,5-bis-(2,2,2-trifluooroethoxy)benzoate (IX), by reaction of 2,5-dihydroxy benzoic acid with 2,2,2-trifluoroethanol perfluorobutanesulphonate in presence of organic bases. This intermediate on subsequent reaction with 2-aminomethylpiperidine gave flecainide. This process again utilizes the costly trifluoroethanol as a leaving group and is hence economically unviable.

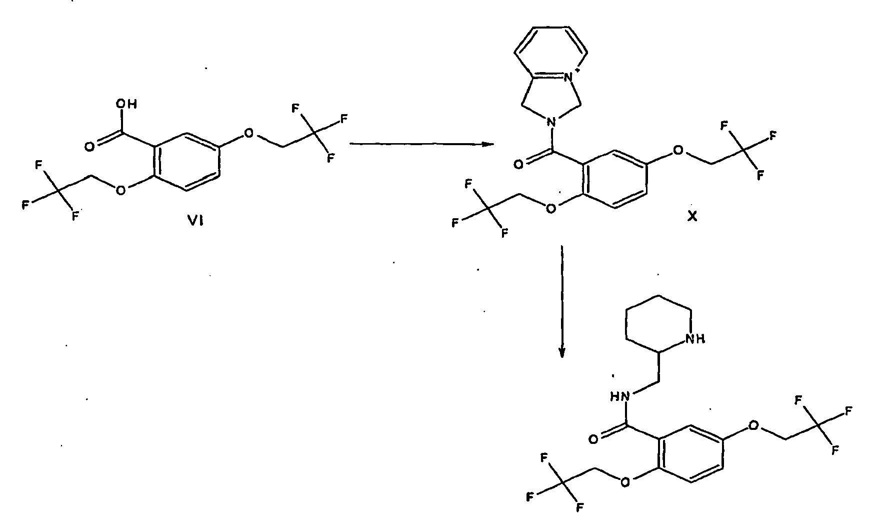

The Spanish patent ES 2007,02 relates to a process for the manufacture of Flecainide comprising of reacting an activated derivative (X) of 2,5-bis(2,2,2-trifluoroethoxy)benzoic acid (X) with 2-azaindolizidine, which is selectively hydrolyzed to flecainide followed by salification with glacial acetic acid. This route again employs a costly chemical for activation and hence not feasible on a commercial scale.

### SUMMARY OF THE INVENTION

An object of the invention is to provide a process for the transformation from a compound of the formula X to a compound of the formula II and thereby to the compound of the formula I in high yields and purities.

Another object of the present invention is to provide a process for the manufacture of the compound of the formula II that is simple, easy and convenient to carry out.

Another object of the invention is to provide a process for the manufacture of the compound of the formula II that is economical and commercially viable. The intermediate can then be subjected to catalytic hydrogenation for the reduction of the pyridyl group to afford flecainide, which is isolated as the acetate.

### DETAILED DESCRIPTION

According to the invention there is provided a process for the manufacture of *N-*(pyridin-2-ylmethyl)-2,5-bis(2,2,2-trifluoroethoxy) benzamide (II) consisting of reacting 2,5-bis (2,2,2-trifluoroethoxy) benzoic acid of the formula VI with an acid chloride of the formula R₁COCl wherein R1 is ethyl, tertiary butyl, or ethoxy in a suitable solvent mixture in the presence of a base at -10 to -50°C to obtain a mixed anhydride of the formula (XI) 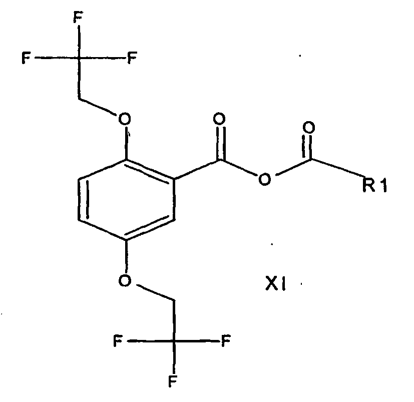 wherein R1 is as defined above which is then condensed with a solution of 2-aminomethyl pyridine at -10 to -40°C followed by hydrolytic workup and extraction of the product and isolation by distillation of the extracting solvent.

As an acid chloride one can utilize methyl chloroformate, ethyl chloroformate, or trimethyl acetyl chloride (pivaloyl chloride) preferably pivaloyl chloride.

As a base one can utilize the tertiary amines such as triethyl amine, pyridine, lutidine or N-methyl morpholine preferably triethyl amine.

The suitable solvent mixture comprises a halogenated hydrocarbon solvent such as methylene chloride, chloroform or ethylene chloride preferably methylene chloride and a polar aprotic solvent selected from the group comprising N,N-dimethyl formamide, N,N-dimethyl acetamide, N,N-dimethyl sulfoxide or N-methyl-2-pyrrolidone preferably N,N-dimethyl acetamide. The halogenated hydrocarbon solvent and polar aprotic solvent are preferably in the ratio 3:1.

The base used for the mixed anhydride formation is employed in the molar ratio from 1:0.9 to 1:1.5 with respect to the compound of the formula VI preferably 1:1.05.

The temperature during the mixed anhydride stage is in the range from -10 to -50°C preferable -20°C.

Typically the condensation reaction of the mixed anhydride with 2-aminomethyl pyridine is conducted from -10 to - 40°C, preferably about -20°C.

The process of the invention does not use any corrosive chemical. The condensation reaction between the mixed anhydride and 2-aminomethyl pyridine is practically quantitative and affords the compound of the formula II in high yields (∼95%) and high purities (∼99%).

Therefore the process of the invention is simple, easy, convenient and fast to carry out. For these reasons the process is economical and commercially viable.

The following examples are illustrative of the invention but not restricted to the scope thereof.

### EXAMPLE-1

60 gm of 2,5-bis (2,2,2-trifluoroethoxy) benzoic acid was suspended in a mixture of 480 ml methylene chloride and 90 ml N,N-dimethyl acetamide. To this 22.9 gm of triethyl amine was added and the mixture was cooled to -30°C. To this 27.4 gm of pivaloyl chloride and the mass stirred at -20 to -30°C. A solution of 21.6 gm of 2-aminomethyl pyridine in 120 ml of methylene chloride was added and the mixture was maintained at -20°C for 120 min. The reaction mixture was quenched in 780 ml of water. After 30 min the lower organic layer was separated and washed with 300 ml 5% sodium carbonate solution. The organic layer was filtered and distilled. The residue was diluted with 360 ml cyclohexane and filtered. The solid was washed with 100 ml cyclohexane and dried at 40°C under vacuum to give 72 gm of *N-*(pyridin-2-ylmethyl)-2,5-bis(2,2,2-trifluoroethoxy) benzamide.
Water (by Karl Fisher)=0.23%. HPLC assay=99.2%. (Theoretical =75 gm)

### EXAMPLE-2

The procedure of example 1 was followed with 26 gm of N-methyl morpholine instead of triethyl amine and the isolated product dried at 40°C under vacuum to give 71 gm of *N-*(pyridin-2-ylmethyl)-2,5-bis(2,2,2-trifluoroethoxy) benzamide.
Water (by Karl Fisher)=0.18%. HPLC assay=99.1%. (Theoretical =75 gm)

### EXAMPLE-3

The procedure of example 1 was followed with 24.7 gm of ethyl chloro formate instead of pivaloyl chloride and the isolated product dried at 40°C under vacuum to give 71.5 gm of *N-*(pyridin-2-ylmethyl)-2,5-bis(2,2,2-trifluoroethoxy) benzamide.
Water (by Karl Fisher)=26%. HPLC assay=99.0%. (Theoretical =75 gm)

### EXAMPLE-4

The procedure of example 1 was followed with 90 ml of N,N-dimethyl formamide instead of N,N-dimethyl acetamide and the isolated product dried at 40°C under vacuum to give 70.8 gm of *N-*(pyridin-2-ylmethyl)-2,5-bis(2,2,2-trifluoroethoxy) benzamide.
Water (by Karl Fisher)=0.29%. HPLC assay=99.1%. (Theoretical =75 gm)

## Claims

1. A process for the manufacture of N-(pyridin-2-ylmethyl)-2,5-bis(2,2,2-trifluoroethoxy)benzamide of the formula II, 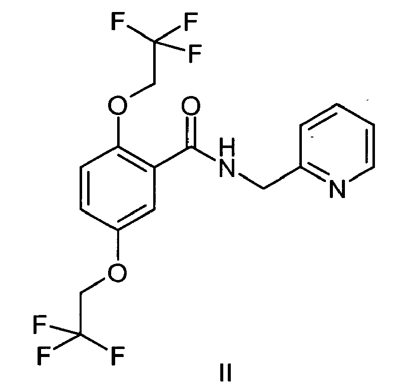 comprising of reacting 2,5-bis(2,2,2-trifluoroethoxy)benzoic acid of the formula VI 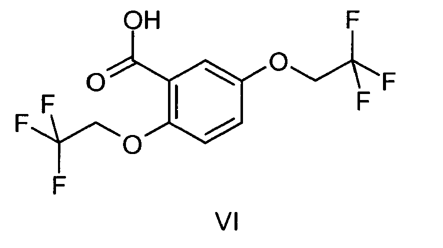 with an acid chloride of the formula R₁COCl wherein R₁ is methyl, ethyl, t-butyl or ethoxy in a solvent mixture, in the presence of a base at -10 to -50°C to obtain a mixed anhydride of the formula XI 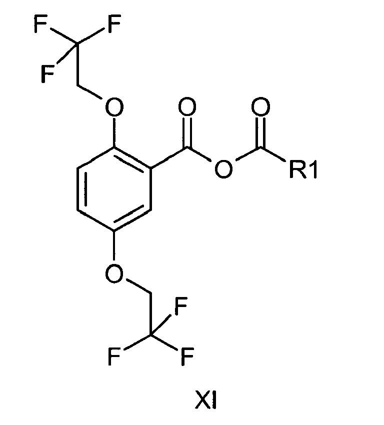 where R₁ is methyl, ethyl, t-butyl or ethoxy, the mixed anhydride is then condensed with a solution of 2-aminomethyl pyridine at -10 to -40°C and subsequently subjected to hydrolytic workup and extraction followed by distillation of the solvent to afford the desired compound of the formula II.

2. The process as claimed in claim 1, wherein the acid chloride can be selected from among the group comprising of methyl chloroformate, ethyl chloroformate and trimethylacetyl chloride (pivaloyl chloride).

3. The process as claimed in claim 2, wherein the acid chloride is trimethylacetyl chloride (pivaloyl chloride).

4. The process as claimed in claim 1, wherein the solvent mixture comprises of a halogenated hydrocarbon solvent such as methylene chloride, chloroform or ethylene chloride and a polar aprotic solvent selected from the group comprising *N*,*N-*dimethylformamide, *N*,*N-*dimethylacetamide, *N*,*N-*dimethylsulfoxide or *N-*methyl-2-pyrrolidone.

5. The process as claimed in claim 4, wherein the solvent mixture comprises of methylene chloride and *N*,*N*-dimethyl acetamide.

6. A process as claimed in claim 1, wherein the base can be selected from the group comprising of tertiary amines such as triethyl amine, pyridine, lutidine or *N-*methyl morpholine.

7. The process according to claim 6, wherein the base is triethylamine.

8. The process as claimed in claim 1, wherein the temperature during the mixed anhydride and condensation stage is -20°C.

9. The process as claimed in claim 1, wherein the base used for the mixed anhydride formation is employed in the molar ratio from 1:0.9 to 1:1.5 with respect to the compound of the formula VI.

10. The process as claimed in claim 9, wherein the molar ratio of the base used for the mixed anhydride formation step is 1:1.05 with respect to the compound of the formula VI.

11. A process as claimed in claim 1, further comprising the conversion of the compound of formula II to a compound of the formula I by catalytic hydrogenation.

## Patentansprüche

1. Verfahren zur Herstellung von N-(Pyridin-2-ylmethyl)-2,5-bis(2,2,2-trifluorethoxy)benzamid der Formel II, 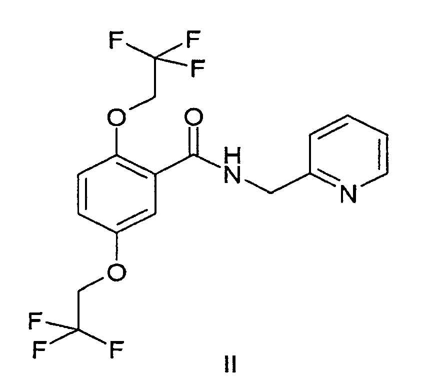 welches umfasst: Umsetzen von 2,5-Bis(2,2,2-trifluorethoxy)benzoesäure der Formel VI 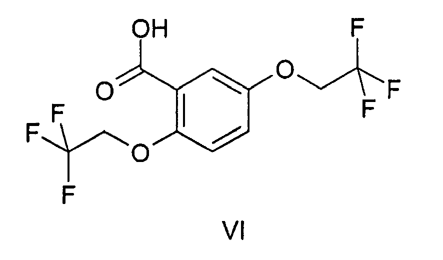 mit einem Säurechlorid der Formel R₁COCl, worin R₁ für Methyl, Ethyl, t-Butyl oder Ethoxy steht, in einem Umsetzungsgemisch in Anwesenheit einer Base bei -10 bis -50°C, um ein gemischtes Anhydrid der Formel XI 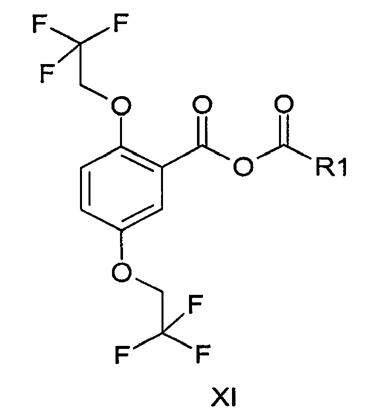 zu erhalten, worin R₁ für Methyl, Ethyl, t-Butyl oder Ethoxy steht, das gemischte Anhydrid dann mit einer Lösung aus 2-Aminomethylpyridin bei -10 bis -40°C kondensiert und nachfolgend hydrolytischer Aufbereitung und Extraktion unterworfen wird,
gefolgt von Destillation des Lösungsmittels, um die gewünschte Verbindung der Formel II zu ergeben.

2. Verfahren wie in Anspruch 1 beansprucht, worin das Säurechlorid ausgewählt werden kann aus der Gruppe, umfassend Methylchlorformat, Ethylchlorformat und Trimethylacetylchlorid (Pivaloylchlorid).

3. Verfahren wie in Anspruch 2 beansprucht, worin das Säurechlorid Trimethylacetylchlorid (Pivaloylchlorid) ist.

4. Verfahren wie in Anspruch 1 beansprucht, worin das Lösungsmittelgemisch ein halogeniertes Kohlenwasserstofflösungsmittel wie Methylenchlorid, Chloroform oder Ethylenchlorid und ein polares aprotisches Lösungsmittel, ausgewählt aus der Gruppe umfassend *N*,*N-*Dimethylformamid, *N*,*N-*Dimethylacetamid, *N,N-*Dimethylsulfoxid oder *N-*Methyl-2-pyrrolidon umfasst.

5. Verfahren wie in Anspruch 4 beansprucht, worin das Lösungsmittelgemisch Methylenchlorid und *N*,*N-*Dimethylacetamid umfasst.

6. Verfahren wie in Anspruch 1 beansprucht, worin die Base ausgewählt werden kann aus der Gruppe, welche tertiäre Amine wie Triethylamin, Pyridin, Lutidin oder *N-*Methylmorpholin umfasst.

7. Verfahren gemäß Anspruch 6, worin die Base Triethylamin ist.

8. Verfahren wie in Anspruch 1 beansprucht, wobei die Temperatur während der gemischten Anhydrid und Kondensationsstufe -20°C ist.

9. Verfahren wie in Anspruch 1 beansprucht, worin die Base, welche für Bildung des gemischten Anhydrids verwendet wird, in einem Molverhältnis von 1:0,9 bis 1:1,5 bezogen auf die Verbindung der Formel VI eingesetzt wird.

10. Verfahren wie in Anspruch 9 beansprucht, worin das Molverhältnis der Base, welche für den Schritt der Bildung des gemischten Anhydrids verwendet wird, 1:1,05 bezogen auf die Verbindung der Formel VI ist.

11. Verfahren wie in Anspruch 1 beansprucht, welches ferner die Umwandlung der Verbindung der Formel II in eine Verbindung der Formel I durch katalytische Hydrierung umfasst.

## Revendications

1. Procédé de fabrication de N-(pyridin-2-ylméthyl)-2,5-bis(2,2,2-trifluoroéthoxy)benzamide de la formule II, 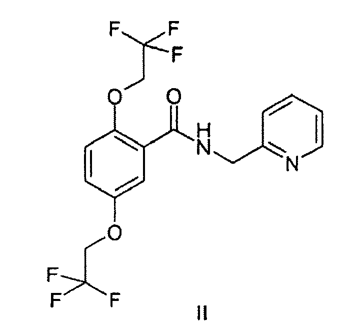 comprenant la réaction d'acide 2,5-bis(2,2,2-trifluoroéthoxy)benzoïque de la formule VI 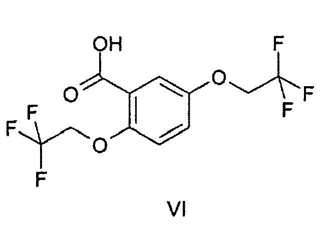 avec un chlorure d'acide de la formule R₁COCl dans laquelle R₁ est un méthyle, éthyle, t-butyle ou éthoxy dans un mélange de solvants, en la présence d'une base à une température de -10 à -50°C pour obtenir un anhydride mixte de la formule XI 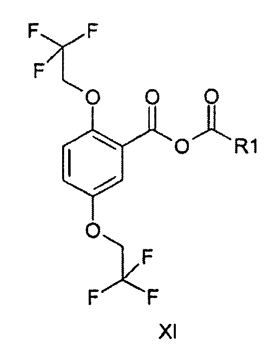 où R₁ est un méthyle, éthyle, t-butyle ou éthoxy, l'anhydride mixte est ensuite condensé avec une solution de 2-aminométhyl pyridine à une température de -10 à -40°C et soumis ensuite à un traitement final et une extraction hydrolytiques suivis par la distillation du solvant pour obtenir le composé souhaité de la formule II.

2. Procédé selon la revendication 1, dans lequel le chlorure d'acide peut être choisi dans le groupe constitué par le chloroformate de méthyle, le chloroformate d'éthyle et le chlorure de triméthylacétyle (chlorure de pivaloyle).

3. Procédé selon la revendication 2, dans lequel le chlorure d'acide est un chlorure de triméthylacétyle (chlorure de pivaloyle).

4. Procédé selon la revendication 1, dans lequel le mélange de solvants comprend un solvant d'hydrocarbure halogéné tel que le chlorure de méthylène, le chloroforme ou le chlorure d'éthylène et un solvant aprotique polaire choisi dans le groupe constitué par N,N-diméthylformamide, N,N-diméthyl-acétamide, N,N-diméthylsulfoxyde ou N-méthyl-2-pyrrolidone.

5. Procédé selon la revendication 4, dans lequel le mélange de solvants comprend du chlorure de méthylène et du N,N-diméthylacétamide.

6. Procédé selon la revendication 1, dans lequel la base peut être choisie dans le groupe constitué par les amines tertiaires telles que l'amine triéthylique, la pyridine, la lutidine ou la N-méthylmorpholine.

7. Procédé selon la revendication 6, dans lequel la base est une triéthylamine.

8. Procédé selon la revendication 1, dans lequel la température pendant l'étape de formation et de condensation d'anhydride mixte est de -20°C.

9. Procédé selon la revendication 1, dans lequel la base utilisée pour la formation d'un anhydride mixte est employée dans le rapport molaire de 1:0,9 à 1:1,5 par rapport au composé de la formule VI.

10. Procédé selon la revendication 9, dans lequel le rapport molaire de la base utilisée pour l'étape de formation d'un anhydride mixte est 1:1,05 par rapport au composé de la formule VI.

11. Procédé selon la revendication 1, comprenant en outre la conversion du composé de formule II en un composé de la formule I par hydrogénation catalytique.
